**Europäisches Patentamt**

**European Patent Office**

**Office européen des brevets**

(19)

(11) Publication number:

**0 124 077**
**B1**

(12) # EUROPEAN PATENT SPECIFICATION

(45) Date of publication of patent specification: **27.09.89**

(51) Int. Cl.⁴: **A 61 K 7/06**

(21) Application number: **84104656.8**

(22) Date of filing: **25.04.84**

(54) Use of a composition as a white hair-preventing agent and kit containing the same.

(30) Priority: **28.04.83 JP 75976/83**

(43) Date of publication of application:
**07.11.84 Bulletin 84/45**

(45) Publication of the grant of the patent:
**27.09.89 Bulletin 89/39**

(84) Designated Contracting States:
**BE DE FR GB IT NL SE**

(56) References cited:
**EP-A-0 076 159**
**FR-A-2 007 366**
**US-A-4 021 538**
**US-A-4 390 341**

**CHEMICAL ABSTRACTS, vol. 55, 1961, page
1901, col. 22602h, Columbus, Ohio, US; R.S.
SNELL et al.: "The effect of large doses of
estrogen and of estrogen and progesterone on
melanin pigmentation", & J. INVEST.
DERMATOL. 35, 73-82**

(73) Proprietor: **Inaba, Masumi**
**3-31-13, Asagayaminami**
**Suginami-ku Tokyo (JP)**

(73) Proprietor: **MEDICAL HAIR RESEARCH
KABUSHIKI KAISHA
3-31-9, Asagaya-Minami
Suginami-ku Tokyo 106 (JP)**

(72) Inventor: **Inaba, Masumi**
**3-31-13, Asagayaminami**
**Suginami-ku Tokyo (JP)**

(74) Representative: **Patentanwälte Popp, Sajda, v.
Bülow, Hrabal & Partner
Widenmayerstrasse 48 Postfach 86 06 24
D-8000 München 86 (DE)**

Courier Press, Leamington Spa, England.

**Description**

The present invention relates to the use of a partly known composition as a white hair-preventive agent, and a kit containing the same.

Human hairs are originally pigmented black, brown, red, golden, etc., depending on genetic inheritance. The color of human hair depends on melanin granules. For example, the coloration of black, brown and golden hairs is said to be determined by the amount of eumelanin pigment granules. Likewise, the coloration of red hair is said to be determined by the amount of pheomelanin pigment granules contained in the hair.

Particles of melanocyte, i.e., a pigment cell having branched projections within the cells of the germinative layer surrounding the dermal-papilla, are interspersed among particles of keratinocytes, i.e., keratin-forming cells, forming the hair cortex. Melanin is secreted from melanocytes to impart the particular color to the hair cortex cells.

Tyrosine, which is one of the amino acids, absorbs enzymes under the action of tyrosinase to be converted into malanin as shown below:

$$\text{tyrosine} \xrightarrow{} \text{dopa} \xrightarrow{} \text{dopa quinone} \underset{\text{(red)}}{\overset{\text{eumelanin (black)}}{\diagdown}}$$

tyrosinase (melanin forming enzyme)

When melanin is not formed, human hairs turn white. Aging and disappearance of melanocytes, metabolic disorder of tyrosine, inactivation of tyrosinase, etc., are thought to stop formation of melanin.

Grey hair represents a reduction in the function of melanocytes, with white hair denoting a complete loss of melanocyte function. Conversion of colored hair to grey hair and then to white hair is irreversible. It is said that white hair can never be replaced by a new growth of the original pigmented hair.

EP—A—0 076 159 discloses a hair growing agent which is to be applied topically and which contains at least one compound selected from stabilized chlorine dioxide, sodium bromate, sodium perborate and potassium bromate, each of these oxidizing compounds in aqueous solution, wherein the stabilized chlorine dioxide content is 100 to 500 ppm, the sodium bromate content is 50.000 to 120.000 ppm, the potassium bromate content is 5 to 12%, and the sodium perborate content is 5%

The object of the present invention is to provide such a composition which will prevent the original pigmented hair from turning white.

Very surprisingly it has been found that this object of the invention can be solved most favorably by the use of the composition as disclosed in EP—A—0 076 159 in combination with a female hormone like estradiol as claimed in claim 1. This composition is able to activate tyrosinase or oxidizing enzyme which serves to promote the formation of melanin within the pigment-forming cells (melanocytes) of the hair follicle.

It is impossible to eliminate white hair if melanocyte function has ceased. However, the present inventor has found that the pigmented hair can be prevented from turning white and growth of pigmented hairs can be promoted by applying a medication containing an oxidizing agent, dopa component and female hormone agent, particularly estradiol, as well as tyrosinase, during the regenerating process of the hair follicle after artificial or natural expiration of a hair. The medication should be applied topically to permeate the hair pore and the cells of the germinative region of the regenerated hair follicle. The oxidizing agent, which serves to promote the activity of tyrosinase, includes potassium bromate, sodium bromate, stabilized chlorine dioxide. Particularly, a synergetic effect is produced if the known oxidizing agent is used together with estradiol, enabling the medication, i.e., the white hair-preventive agent, to produce significant effects.

The normal pigmented hair has a regenerating cycle of 6 to 7 years, whereas the regenerating cycle of white hair is shorter than that of the pigmented hair. Thus, if the white hair-preventive agent is used over a long period of time, the entire hair is finally pigmented completely because the activated tyrosinase serves to encourage growth of the original pigmented hair in the regenerating phase. If the white hair-preventive agent is applied while employing an artificial hair-regenerating measure such as brushing, the white hair problem can be solved in a shorter period. The white hair-preventive agent of the present invention is particularly effective if applied in the early stage of white hair occurrence. As can be assumed from the disclosure in EP—A—0 076 159 the agent of the present invention also serves to promote new growth of hair.

The amounts of the oxidizing agent and estradiol used in the present invention should be large enough to promote activation of tyrosinase or oxidizing enzyme serving to promote the melanin formation within the pigment-forming cell (melanocyte). However, the amounts mentioned should not be large enough to have negative side effects. The mixture of the oxidizing agent and estradiol, which may contain additional components, as required, is applied to the scalp skin and then the scalp is lightly massaged. This treatment is repeated several times a day and continued over a considerable period of time. Before or after the application of the oxidizing agent-containing solution, a solution containing tyrosine or an intermediate material to be produced in the process of forming melanin such as dopa, dopa quinone and hallachrome may preferably be applied to accelerate melanin formation. Thus, the invention also refers to a kit containing the white hair preventing agent of the invention together with a solution containing tyrosine or an intermediate material as defined above.

Tyrosine or the intermediate materials mentioned above may be applied simultaneously with the oxidizing agent-containing solution.

The term "stabilized chlorine dioxide" mentioned previously represents an aqueous solution of chlorine dioxide subjected to a chemical stabilizing treatment which is disclosed in, for example, U.S. Patent Nos. 4,296,103; 3,147,124; 3,123,521; and 2,701,781. In order to chemically stabilize chlorine dioxide, which is otherwise explosive, a stabilizer such as caustic soda is added to chlorine dioxide so as to obtain the stabilized chlorine dioxide in question. It is known that stabilized chlorine dioxide can be made harmless to the human body by properly adjusting the chlorine dioxide concentration of the aqueous solution or by dissolving suitable additives in the solution.

Clinical tests were conducted using stabilized chlorine dioxide (in aqueous solution), aqueous solutions of sodium bromate, sodium perborate, potassium bromate, and a female hormone agent. In each test, the medication was applied to three oriental men, 35 to 50 years-old, 3 times a day at intervals of 5 hours. The stabilized chlorine dioxide used in the test was an aqueous solution containing about 25% of sodium chlorite and 4 to 5% of caustic soda used as a stabilizer. The original solution with chlorine dioxide concentration of 5 to 15% was properly diluted for actual application.

Table 1 shows the results of the tests. The average evaluations are shown in the table because the results of the test were somewhat different from person to person.

As seen in Table 1, the white hair-preventive agent of the present invention suppresses most favorably white hair (see test no. 15).

## Table 1

| Test No. | Chemicals (Conc.) | Effect (Condition of Regen. Hair) | |
| --- | --- | --- | --- |
| | | 6 Months Later | 12 Months Later |
| 1 | A*(50 ppm) | Grey hair | Fairly black |
| 2 | A (100 ppm) | Grey & black mixed | Ditto |
| 3 | A (250 ppm) | Somewhat black | Ditto |
| 4 | A (500 ppm) | ** | *** |
| 5 | B*(5%) | No effect | No effect |
| 6 | B (8%) | Grey hair | Fairly black |
| 7 | B (12%) | Ditto | Ditto |
| 8 | C*(5%) | Grey hair | Grey hair |
| 9 | C (8%) | Tends to turn brown | Tend to turn brown |
| 10 | C (12%) | Tends to break when combed | Tend to break when combed |
| 11 | D*(5%) | No effect | No effect |
| 12 | D (8%) | Grey hair | Grey hair |
| 13 | D (12%) | Ditto | Ditto |
| 14 | E* | Somewhat black | About 50% black |
| 15 | F* | Clearly blackened | Blackening is prominent |

# EP 0 124 077 B1

Note:

* A: Stabilized chlorine dioxide (aqueous solution)
B: Aqueous solution of sodium bromate
C: Aqueous solution of sodium perborate
D: Aqueous solution of potassium bromate
E: Only female hormone agent (estradiol), 0.05 mg/cc
F: Mixture of estradiol (0.05 mg/cc) and chlorine dioxide (250 ppm)
** ... Blackened, but the regenerated hairs tend to turn brown because the oxidizing action is too strong.
*** ... The regenerated hairs tend to break off when combed because the oxidizing action is too strong.

### Claims

1. Use of a composition as a white hair preventing agent which is to be applied topically and which contains at least one compound selected from stabilized chlorine dioxide, sodium bromate, sodium perborate and potassium bromate, each of these oxidizing compounds being in aqueous solution, wherein the stabilized chlorine dioxide content is 100 to 500 ppm, the sodium bromate content is 50.000 to 120.000 ppm, the potassium bromate content is 5 to 12% and the sodium perborate content is 5%, and which further contains estradiol in alcoholic solution, in particular in a 60% alcoholic solution at a rate of 0,05 to 0,10 mg/cm³.

2. Use of the composition according to claim 1 together with tyrosine or an intermediate material to be produced in the process of forming melanin, namely dopa, dopa quinone and hallachrome.

3. A kit comprising the composition according to claim 1 or 2 for the use of suppressing conversion of pigmented hair to white hair.

### Patentansprüche

1. Verwendung einer Zusammensetzung als Mittel zur Verhinderung von weißen Haaren, das äußerlich anwendbar ist und wenigstens eine Verbindung enthält, die stabilisiertes Chlordioxid, Natriumbromat, Natriumperoxoborat oder Kaliumbromat ist, wobei jede dieser oxidierenden Verbindung in wäßriger Lösung vorliegt und der Gehalt an stabilisiertem Chlordioxid 100—500 ppm, der Gehalt an Natriumbromat 50,000—120,000 ppm, der Gehalt an Kaliumbromat 5—12% und der Gehalt an Natriumperoxoborat 5% beträgt, wobei die Zusammensetzung außerdem Östradiol in alkoholischer Lösung, insbesondere in einer 60% alkoholischen Lösung in eine Konzentration von 0,05—0,10 mg/cm³, enthält.

2. Verwendung der Zusammensetzung nach Anspruch 1 gemeinsam mit Tyrosin oder einem Zwischenprodukt, das bei der Melaninbildung erzeugt wird, und zwar Dopa, Dopachinon und Hallachrom.

3. Eine die Zusammensetzung nach Anspruch 1 oder 2 enthaltende Packung zur Verwendung bei der Unterdrückung der Umwandlung von pigmentiertem Haar in weißes Haar.

### Revendications

1. Utilisation d'une composition comme agent de prévention des cheveux blancs, destinée à être utilisée en topique et qui contient au moins un composé choisi parmi le dioxyde de chlore stabilisé, le bromate de sodium, le perborate de sodium et le bromate de potassium, chacun de ces composants oxydants étant en solution aqueuse, la teneur en dioxyde de chlore stabilisé étant de 100 à 500 ppm, la teneur en bromate de sodium étant de 50 000 à 120 000 ppm, la teneur en bromate de potassium étant de 5 à 12% et la teneur en perborate de sodium étant de 5%, et qui contient en outre de l'oestradiol en solution alcoolique, en particulier en solution alcoolique à 60% à une concentration de 0,05 à 0,10 mg/cm³.

2. Utilisation de la composition conforme à la revendication 1, conjointement avec de la tyrosine ou une substance intermédiaire produite dans le procédé de formation de la mélanine, à savoir le dopa, la dopa-quinone et le hallachrome.

3. Trousse comprenant la composition conforme à la revendication 1 ou 2, à utiliser pour supprimer la conversion de cheveux pigmentés en cheveux blancs.